# EUROPÄISCHE PATENTSCHRIFT

(11) **EP 3 843 622 B1**
(45) Veröffentlichungstag und Bekanntmachung des Hinweises auf die Patenterteilung: **05.07.2023**
(21) Anmeldenummer: 19762740.9
(22) Anmeldetag: 27.08.2019
(51) Int. Cl.: A61B 5/022, A61B 5/024, A61B 5/0225

(54) **MEHRTEILIGE VORRICHTUNG ZUM NICHT-INVASIVEN ERFASSEN VON VITALPARAMETERN**
MULTI-PART APPLIANCE FOR NON-INVASIVE DETECTION OF VITAL PARAMETERS
DISPOSITIF EN PLUSIEURS PARTIES PERMETTANT D'ACQUÉRIR DES PARAMÈTRES VITAUX DE FAÇON NON INVASIVE

(30) Priorität: 29.08.2018 DE 102018006846
(43) Veröffentlichungstag der Anmeldung: 07.07.2021
(73) Patentinhaber: Pulsion Medical Systems SE, 85622 Feldkirchen (DE)
(72) Erfinder: WEBER, Aaron, 85570 Markt Schwaben (DE); HEIN, André, 73728 Esslingen am Neckar (DE)
(74) Vertreter: Kehl, Ascherl, Liebhoff & Ettmayr Patentanwälte Partnerschaft mbB
(86) Internationale Anmeldenummer: PCT/EP2019/072848
(87) Internationale Veröffentlichungsnummer: WO 2020/043726

(56) Entgegenhaltungen:
- US-A- 5 735 798
- US-A1- 2015 038 859
- US-A1- 2018 235 478

## Beschreibung

Die Erfindung betrifft eine Vorrichtung zur Vitalparametererfassung, insbesondere zur kontinuierlichen Bestimmung des intra-arteriellen Blutdruckes an zumindest einem Finger einer Hand, mit Hilfe eines Fingersensors. Die Vorrichtung basiert auf dem Zusammenwirken einer Basiseinrichtung und einer daran über eine spezielle Schnittstelle angekoppelten Körperkontakteinrichtung. Die Vorrichtung nutzt bevorzugt eine plethysmographische Anordnung, mit zumindest einer Lichtquelle, zumindest einem Lichtaufnehmer bzw. Lichtdetektor und zumindest einer aufblasbaren Manschette.

Die kontinuierliche nicht-invasive Messung des Blutdruckes stellt bis heute eine große Herausforderung an die Messtechnik dar. Am Markt beginnt sich die sog. "Vascular Unloading Technique" durchzusetzen, die auf eine Publikation von Peňáz (Digest of the 10th International Conference on Medical and Biological Engineering 1973 Dresden) zurückgeht und durch verschiedene Elemente verbessert wurde.

Bei der Vascular Unloading Technique wird nahinfrarotes Licht in einen Finger eingestrahlt, und anhand des mittels eines Photodetektors aufgefangenen nicht absorbierten Anteils der pulsatile (pulsförmige) Blutfluss (tatsächlich das sich verändernde Blutvolumen) im Finger bestimmt. Für dieses, auch Photoplethysmographie (PPG) genannte, Verfahren wird das (nahinfrarote) Licht üblicherweise mit Hilfe von einer oder mehreren Leuchtdioden (LED), die mit einer oder mehreren Wellenlängen arbeiten, erzeugt sowie mit Hilfe von einer oder mehreren lichtempfindlichen Empfängerdioden (Photodioden) detektiert. Anstelle von Dioden sind auch andere Arten von Photoempfängern grundsätzlich geeignet.

Ein Regelungssystem hält nun den plethysmographisch registrierten Fluss (bzw. das detektierte Blutvolument) und somit das resultierende photoplethysmographische Signal (Volumssignal v(t)) konstant, in dem ein Gegendruck in einer Manschette (Cuffpressure) pc(t) am Finger aufgebracht wird. Dieser Gegendruck pc(t) wird dabei üblicherweise von einem schnellen Ventil oder Ventilsystem im Zusammenspiel mit einer Pumpe geregelt. Die diesbezügliche Ansteuerung des Ventils bzw. des Ventilsystems wird von einer Regelungseinheit durchgeführt, die vorzugweise mit einem Microcomputer realisiert wird. Die wesentlichen Eingangssignale sind dabei das PPG-Signal v(t) und der Manschettendruck pc(t). Der für die Konstanthaltung des PPG-Signales v(t) notwendige Druck pc(t) entspricht nun dem intra-arteriellen Blutdruck pa(t).

Dafür ist es erforderlich, dass sich der Manschettendruck pc(t) mindestens so schnell verändern lässt, wie sich auch der intra-arterielle Blutdruck pa(t) ändert, damit die Echtzeitbedingung erfüllt ist. Die obere Grenzfrequenz von pa(t) und somit die höchste Druckänderungsgeschwindigkeit liegt oberhalb von zumindest 20Hz, was für ein Druckregelsystem durchaus eine Herausforderung darstellt. Hieraus folgt, dass sich die Druckregelung durch ein Ventil bzw. Ventilsystem vorteilhafterweise in unmittelbarer Nähe der Manschette befindet. Bei zu langen Luftleitungen droht der Verlust dieser Grenzfrequenzbedingung wegen der Tiefpasswirkung der Leitungen.

Zahlreiche Veröffentlichungen betreffend die Vascular Unloading Technique sind bekannt:
Aus der US 4,406,289 ist ein mechanisches Ventil bekannt, das den Gegendruck in der Fingermanschette mit der gewünschten Genauigkeit regelt, wenn es mit einer linear arbeitenden Pumpe versorgt wird. Das Ventil ist in einem Gehäuse am distalen Unterarm untergebracht und versorgt so die Fingermanschette über einen kurzen Schlauch mit dem Druck pc(t).

Die US 4,524,777 beschreibt ein Druckerzeugungssystem für die Vascular Unloading Technique, wobei ebenfalls ein konstanter Manschettendruck Pc mit einer linearen Pumpe erzeugt wird, der mit Druckschwankungen Δpc(t) aus einem parallel geschalteten "Shaker" bzw. einem " Driving Actuator" überlagert wird.

In US 4,726,382 ist eine Fingermanschette für die Vascular Unloading Technique offenbart, die Schlauchanschlüsse für die Versorgung mit dem Manschettendruck pc(t) besitzt. Die Länge der Luftschläuche reicht bis zum Druckerzeugungssystem, das wiederum am distalen Unterarm befestigt ist.

Die WO 2000/059369 A1 beschreibt ebenfalls ein Druckerzeugungssystem für die Vascular Unloading Technique. Das Ventilsystem besteht hier aus einem separaten Einlass- und einem separaten Auslassventil. Während bei den Patentschriften US 4,406,289 und US 4,524,777 eine relativ lineare Proportionalpumpe verwendet werden muss, erlaubt dieses System die Verwendung von einfachen kostengünstigen Pumpen, da störende Oberwellen durch die Anordnung der Ventile eliminiert werden können. Ferner kann der Energieverbrauch der einfachen Pumpe durch das Ventilprinzip wesentlich reduziert werden.

Aus der WO 2004/086963 A1 ist ein System für die Vascular Unloading Technique bekannt, bei dem in einem Finger kontinuierlich der Blutdruck bestimmt werden kann, während im benachbarten Finger eine Kontrolle der Messqualität vorgenommen wird ("Watch Dog"-Funktion). Nach einer Zeit wechselt das System automatisch den "Messfinger" mit dem "Überwachungsfinger".

US 2015/0038859 A1 offenbart eine aufblasbare Manschettenvorrichtung für den Einsatz in einer Blutdrucküberwachungsvorrichtung, wobei der Manschettendruck mittels einer in eine Ventilkammeranordnung einzusetzende Gaspatrone aufgebracht wird.

US 2018/0235478 A1 beschreibt im Zusammenhang mit einem hochvernetzten System verteilter Einrichtungen zum Messen und Auswerten physiologischer Parameter eine Fingermanschetteneinheit. Der aufblasbare Manschettenteil ist abnehmbar und auswechselbar.

Aus US 5,735,798 ist ein in einen Toilettensitz eingebautes Sphygmomanometer mit einer einziehbaren Manschettenvorrichtung bekannt.

Die WO 2005/037097 A1 beschreibt ein Regelungssystem für die Vascular Unloading Technique mit mehreren ineinander verflochtene Regelkreisen.

Die WO 20101050798 A1 offenbart ein am distalen Unterarm befestigtes Druckerzeugungssystem ("Frontend") mit nur einem Ventil, an dem eine Fingermanschette für die Vascular Unloading Technique angebracht werden kann.

Bei einem in WO 2011/045138 A1 beschriebenen Druckerzeugungssystem für die Vascular Unloading Technique wird - ähnlich wie aus der WO 2000/059369 bekannt - die Energieaufnahme der Pumpe reduziert, und es können Oberwellen eliminiert werden.

Die WO 2011/051819 A1 offenbart eine mittels digitaler Elektronik verbesserte Implementierung der Vascular Unloading Technique zur Erhöhung der Stabilität sowie zur weiteren Miniaturisierung.

In WO 2011/051822 A1 ist ein Verfahren für die Vascular Unloading Technique beschrieben, bei dem die gemessenen Signale v(t) und pc(t) zur Erhöhung langfristiger Stabilität und zum Ermitteln weiterer hämodynamischer Parameter verarbeitet werden. Insbesondere werden ein Verfahren zur Elimination von Effekten, die von vasomotorischer Veränderungen der Fingerarterien herrühren, sowie eine Methode zur Bestimmung des Herzzeitvolumens (Cardiac Ouput CO) offenbart.

Die WO 2012/032413 A1 beschreibt neuartige Fingersensoren, die ein Wegwerfteil (Disposable) zur einmaligen Verwendung besitzen. Dabei ist die mit dem Finger in Berührung kommende Manschette aus hygienischen Gründen im Wegwerfteil untergebracht, wohingegen das zugehörige Druckerzeugungs- und Druckregelsystem in einem wiederverwertbaren Teil untergebracht ist. Entsprechend ist hier eine trennbare pneumatische Verbindung zwischen Wegwerfteil und wiederverwertbarem Teil vorzusehen.

In der Regel wird das Druckerzeugungs- und Druckregelsystem im Stand der Technik am distalen Unterarm, proximal des Handgelenkes, angebracht, was wesentliche Nachteile mit sich bringt: Diese Stelle wird häufig für intravenöse Zugänge verwendet und auch der intraarterielle Zugang am distalen Ende der Radialerterie sollte für Notfälle frei sein. Derartige Zugänge können durch das Druckerzeugungs- und Druckregelsystem und dessen Befestigung blockiert werden. Außerdem kann im Betrieb das System verrutschen bzw. kippen. Dies kann sich nachteilig auf den Sitz der Sensoren auswirken. Der Sitz der Sensoren würde sich außerdem verbessern, wenn sich der zu messende Finger bzw. die entsprechende Hand in einer gewissen Ruhelage befindet.

Zur Überwindung dieser Problematik schlägt die Druckschrift WO 20171143366 A1 ein Messsystem zur kontinuierlichen Bestimmung des intra-arteriellen Blutdruckes an zumindest einem Finger einer Hand, mit zumindest einem Fingersensor, mit einem plethysmographischen System, mit zumindest einer Lichtquelle, vorzugsweise LED, mit einer oder mehreren Wellenlängen und zumindest einem Lichtaufnehmer und zumindest einer aufblasbaren Manschette, sowie mit einem Druckerzeugungssystem mit zumindest einem mit Hilfe des plethysmographischen Systems in Echtzeit geregelten Ventil zur Erzeugung eines Druckes in der Manschette, der im Wesentlichen dem intra-arteriellen Blutdruck im Finger entspricht, vor, wobei das Messsystem ein Gehäuse mit einer Oberfläche aufweist, die als Auflagefläche für den zumindest einen Finger und die angrenzenden Bereiche der Handinnenfläche dient. Die Hand ruht hier also auf einer Auflage, unter der sich wesentliche Komponenten befinden, die bei herkömmlichen Systemen am Unterarm befestigt waren.

Ähnlich der oben erwähnten WO 2012/032413 A1 ist die Manschette in einem vom Gehäuse (und somit von der Handauflage) trennbaren Wegwerfteil untergebracht. Entsprechend ist wiederum eine trennbare pneumatische Verbindung zwischen Wegwerfteil und wiederverwertbarem Teil vorzusehen.

Die pneumatische Verbindung in den letztgenannten Druckschriften wirft das Problem auf, dass insbesondere am wiederverwertbaren Gehäuse Öffnungen vorliegen, die ggf. schwierig zu reinigen und zu desinfizieren sind.

Es ist daher das Ziel der vorliegenden Erfindung eine Vorrichtung zur nichtinvasiven Erfassung von Vitalparametern bereitzustellen, die eine verbesserte Reinigung und Desinfektion ermöglicht.

Die zuvor genannte Aufgabe wird erfindungsgemäß durch eine Vorrichtung zum Erfassen von Vitalparametern, insbesondere des arteriellen und/oder venösen Blutdrucks, gemäß Anspruch 1 gelöst. Diese Vorrichtung kann auch als Messsystem bezeichnet werden und weist bevorzugt mindestens eine Basiseinrichtung mit Mitteln zur Druckbeaufschlagung eines Arbeitsfluids und eine Körperkontakteinrichtung zum definierten Anordnen eines Körperteils, insbesondere eines Fingers oder mehrerer Finger, auf. Die Körperkontakteinrichtung kann als Fingersensor oder Fingerpositioniereinrichtung oder als Wechselteil bezeichnet werden. Die Basiseinrichtung und die Körperkontakteinrichtung sind dabei zum Ausbilden einer Betriebskonfiguration lösbar miteinander koppelbar, wobei die Basiseinrichtung mindestens ein Gehäuse zum Begrenzen eines Aufnahmeraums aufweist, wobei das Gehäuse zumindest einerseits eine Kontaktoberfläche zur Anordnung der Körperkontakteinrichtung aufweist, wobei die Kontaktoberfläche eine mittels eines Verschlussteils einer Ventileinrichtung verschließbare Durchgangsöffnung aufweist, wobei die Ventileinrichtung zumindest überwiegend im Inneren des Aufnahmeraums angeordnet ist. Die Körperkontakteinrichtung ist somit an das Gehäuse und dadurch an die Basiseinrichtung ankoppelbar. Bevorzugt ist eine Feder, insbesondere aus Metall oder einem Polymermaterial, zum Federkraftbeaufschlagen des Verschlussteils vorgesehen. Bei dem Arbeitsfluid handelt es sich in der Regel um ein Gas, beispielsweise Luft, allerdings sind auch Umsetzungen mit einer Flüssigkeit als Arbeitsfluid vorteilhaft möglich. Als Fluid wird in der vorliegenden Anmeldung gemäß dem üblichen Wortsinn generell ein Gas oder eine Flüssigkeit verstanden. Die Feder kann hierbei eine Zugfeder oder eine Druckfeder sein, wobei die Feder bevorzugt eine Schraubenfeder ist. Das Verschlussteil ist durch die Feder in eine die Durchgangsöffnung verschließende Position überführbar und die Durchgangsöffnung ist Bestandteil einer basiseinrichtungsseitigen Fluidleitung. Die Fluidleitung ist hierbei bevorzugt zum Leiten eines gasförmigen Arbeitsfluids, insbesondere Luft, ausgebildet. Die Körperkontakteinrichtung weist mindestens einen mit dem Arbeitsfluid beaufschlagbaren Druckapplikator, insbesondere für einen oder zwei Finger, zur gesteuerten Druckbeaufschlagung des Körperteils, insbesondere bei mehreren Fingern, jeden der Finger bevorzugt einzeln, auf. Die Körperkontakteinrichtung weist bevorzugt mindestens ein Dichtungselement zum Erzeugen einer Fluidverbindung mit der Basiseinrichtung auf. Das Dichtungselement weist bevorzugt ein oder mindestens ein Polymermaterial auf oder besteht aus einem Polymermaterial. Bevorzugt weist das Dichtungselement einen Fixieranteil zur Anordnung an einer Aufnahmestelle der Körperkontakteinrichtung und einen Dichtanteil auf. Der Fixieranteil und der Dichtanteil sind bevorzugt einstückig miteinander verbunden, wobei der Fixieranteil und der Dichtanteil aus unterschiedlichem Materialien bestehen können (z.B. zwei oder mehrere Komponentenanteile). Der Fixieranteil ist bevorzugt zum Umschließen der Aufnahmestelle ausgebildet und erstreckt sich bevorzugt hohl oder rohrförmig in Längsrichtung des Dichtungselements. Die Körperkontakteinrichtung zwischen dem Dichtungselement und dem Druckapplikator weist bevorzugt eine körperkontakteinrichtungsseitige Fluidleitung auf, wobei das Dichtungselement zum Umschließen der Durchgangsöffnung an die Kontaktoberfläche der Basiseinheit anpressbar ist. Die Körperkontakteinrichtung weist bevorzugt eine zapfenartige, insbesondere hervorstehende, Auslenkeinrichtung, zum Auslenken des Verschlussteils auf. Die Auslenkeinrichtung besteht bevorzugt aus einem Polymermaterial oder weist ein Polymermaterial auf, sie kann aber vorteilhaft auch metallisch oder metallverstärkt ausgeführt werden. Die Auslenkeinrichtung lenkt in der Betriebskonfiguration das Verschlussteil entgegen der Federkraft und unter Ausbildung der Fluidverbindung aus.

Diese Lösung ist vorteilhaft, da die Körperkontakteinrichtung und die Basiseinrichtung als getrennte Einrichtungen ausgeführt werden und somit unterschiedliche Funktionen erfüllen können. Bevorzugt ist die Basiseinrichtung wiederverwendbar und die Oberfläche der Basiseinrichtung sterilisierbar bzw. desinfizierbar. Die Körperkontakteinrichtung kann ebenfalls wiederverwendbar ausgeführt werden. Alternativ kann die Körperkontakteinrichtung zur Einmalverwendung bestimmt sein. Bevorzugt weist die Körperkontakteinrichtung keine für die Messung bzw. Vitalparametererfassung erforderlichen elektrischen Bauteile auf, diese sind bevorzugt in der Basiseinrichtung angeordnet oder Bestandteil davon.

Weitere bevorzugte Ausführungsformen sind Gegenstand der Unteransprüche und der nachfolgenden Beschreibungsteile.

Das Verschlussteil bildet gemäß einer weiteren bevorzugten Ausführungsform der vorliegenden Erfindung einen Teil der Kontaktoberfläche des Gehäuses aus, wobei der durch das Verschlussteil ausgebildete Teil der Kontaktoberfläche in einer entkoppelten Konfiguration (bzw. Nicht-Betriebskonfiguration) bündig mit den umliegenden Oberflächenanteilen der Kontaktoberfläche abschließt. Diese Ausführungsform ist vorteilhaft, da dadurch eine einfache und gründliche Reinigung der Oberfläche der Basiseinrichtung möglich wird. Bündig abschließen bedeutet hierbei bevorzugt, dass die Kontaktoberfläche am Übergang zwischen Verschlussteil und Gehäuse keine Vor- oder Rücksprünge aufweist.

Bevorzugt weist der Oberflächenanteil der Kontaktoberfläche im Radius von bis zu 10mm oder von bis zu 20mm oder von bis zu 30 mm oder von bis zu 40mm oder von bis zu 50mm um das axiale Zentrum des Verschlussteils herum kein Loch oder Löcher, Vertiefung/en und/oder Rille/n auf. Bevorzugt trifft dies für die gesamte Kontaktoberfläche oder Gesamtoberfläche der Basiseinrichtung zu. Bevorzugt weist die Kontaktoberfläche einen mittleren Rauheitswert von <5µm oder <1µm oder <0,5µm oder <0.1µm oder <0,05µm oder <0,01 µm auf.

Das Dichtungselement weist gemäß einer weiteren bevorzugten Ausführungsform der vorliegenden Erfindung einen konischen oder glockenförmigen Kragen auf, wobei der konische oder glockenförmige Kragen die Auslenkeinrichtung zumindest zeitweise und zumindest abschnittsweise in Umfangsrichtung umschließt, wobei die Auslenkeinrichtung im Zentrum des konischen oder glockenförmigen Kragens angeordnet ist, wobei der konische oder glockenförmige Kragen im Bereich eines ersten Endes in radialer Richtung weiter von der Auslenkeinrichtung beabstandet ist als im Bereich eines zweiten Endes, wobei das erste Ende des konischen oder glockenförmigen Kragens zum Kontaktieren der Kontaktoberfläche bestimmt ist. Diese Ausführungsform ist vorteilhaft, da durch den konischen oder glockenförmigen Kragen eine leichte Anbringung der Körperkontakteinrichtung an der richtigen Position an der Basiseinrichtung unterstützt wird. Ferner ergibt sich vorteilhaft ein Toleranzausgleich in axialer und radialer Richtung.

Die Auslenkeinrichtung ist gemäß einer weiteren bevorzugten Ausführungsform der vorliegenden Erfindung fix gegenüber dem Dichtungselement positioniert, und zwischen dem konischen oder glockenförmigen Kragen des Dichtungselements und der Auslenkeinrichtung ist bevorzugt mindestens oder genau ein Fluiddurchlass ausgebildet, bevorzugt sind mehrere Fluiddurchlässe ausgebildet. Der Fluiddurchlass oder die Fluiddurchlässe sind dabei bevorzugt als axiale Löcher oder Bohrungen zu verstehen.

Die Auslenkeinrichtung ist gemäß einer weiteren bevorzugten Ausführungsform der vorliegenden Erfindung gegenüber dem Dichtungselement beweglich angeordnet, wobei das Dichtungselement eine Durchgangsöffnung ausbildet, wobei die Durchgangsöffnung fluiddicht durch die Auslenkeinrichtung verschließbar ist, wobei die Auslenkeinrichtung die Durchgangsöffnung des Dichtungselements in einer Nichtgebrauchskonfiguration verschließt und in der Betriebskonfiguration frei gibt. Diese Ausführungsform ist vorteilhaft, da die Gefahr einer Kontamination der in der Körperkontakteinrichtung ausgebildeten Arbeitsfluidleitungskomponenten und/oder Arbeitsfluidaufnahmekomponenten durch biologisches Material, wie Bakterien, Viren oder Pilze, reduziert bzw. begrenzt wird. Auch eine Abdichtung gegen das Eindringen von Reinigungs- bzw. Desinfektionsmittel kann so vorteilhaft erzielt werden.

Gemäß einer weiteren bevorzugten Ausführungsform der vorliegenden Erfindung ist eine Feder zum Auslenken der Auslenkeinrichtung vorgesehen, wobei die Auslenkeinrichtung bevorzugt geradlinig auslenkbar ist, wobei die Feder in der Betriebskonfiguration infolge der geradlinigen Auslenkung der Auslenkeinrichtung bevorzugt stärker gestaucht ist als in der Nichtgebrauchskonfiguration. Diese Ausführungsform ist vorteilhaft, da die Fluidverbindung zwischen der Körperkontakteinrichtung und der Basiseinrichtung selbsttätig und erst bei der mechanischen Kopplung der beiden Einrichtungen miteinander erzeugt wird.

Die Auslenkung der Auslenkeinrichtung bzw. des Auslenkelements ist gemäß einer weiteren bevorzugten Ausführungsform der vorliegenden Erfindung durch einen Anschlag begrenzt, wobei die Durchgangsöffnung infolge der Verformung des konischen oder glockenförmigen Kragens betragsmäßig näher an die Basiseinrichtung heranbewegbar ist als das Auslenkelement auslenkbar ist oder als das Verschlussteil der Ventileinrichtung der Basiseinrichtung ausgelenkt wird oder auslenkbar ist. Bevorzugt ist die Federkraft der das Verschlussteil beaufschlagenden Feder von der Federkraft der das Auslenkelement beaufschlagenden Feder verschieden. Bevorzugt höher oder niedriger. Alternativ können die Federkräfte auch gleich groß sein. Diese Ausführungsform ist vorteilhaft, da die Erzeugung der Fluidverbindung bei unterschiedlichen Federkräften eine zeitlich versetzte Freigabe der einzelnen Leitungsabschnitte bewirkt. Die Auslenkung des Auslenkelements und die Auslenkung des Verschlussteils der Ventileinrichtung der Basiseinrichtung entspricht gemäß einer weiteren bevorzugten Ausführungsform der vorliegenden Erfindung in Summe der sich infolge der Verformung des konusförmigen Kragens resultierenden Annäherung der Durchgangsöffnung des Dichtungselements an die Kontaktoberfläche.

Gemäß einer weiteren bevorzugten Ausführungsform der vorliegenden Erfindung ist basiseinrichtungsseitig mindestens eine Pumpeinrichtung zum Druckbeaufschlagen des Arbeitsfluids, insbesondere Luft, vorgesehen, wobei die Pumpeinrichtung bevorzugt unmittelbar mit der Fluidleitung gekoppelt ist. Alternativ ist es möglich, eine externe Pumpeinrichtung vorzusehen, die mit der Basiseinrichtung verbindbar ist.

Bevorzugt ist mindestens eine optische Signalquelle und eine optische Sensoreinrichtung als Bestandteil des Gehäuses oder innerhalb des Gehäuses vorgesehen, wobei die Pumpeinrichtung oder der Druckapplikator oder einer in der Fluidleitung vorgesehenen, insbesondere regelbaren bzw. ansteuerbaren, Ventileinrichtung in Abhängigkeit von optischen Signalen regelbar ist, welche die optische Sensoreinrichtung erfasst. Der Druckapplikator kann hierbei eine ansteuerbare bzw. regelbare Ventileinrichtung aufweisen oder eine ansteuerbare bzw. regelbare Ventileinrichtung kann Bestandteil der Fluidleitung oder der Pumpeinrichtung sein. Die Ventileinrichtung bzw. Druckeinstellventileinrichtung kann dabei zur Einstellung eines konstanten Drucks oder einer vorgegebenen bzw. vorgebbaren Druckkurve ansteuerbar sein. Insbesondere ist die Ventileinrichtung bzw. Druckeinstellventileinrichtung elektrisch ansteuerbar. Die Ventileinrichtung bzw. Druckeinstellventileinrichtung ist bevorzugt in der Basiseinrichtung oder in der Körperkontakteinrichtung angeordnet.

Die oben genannte Aufgabe wird ebenfalls durch eine Basiseinrichtung zur Verwendung in einer hierin beschriebenen erfindungsgemäß Vorrichtung gelöst. Die Basiseinrichtung weist dabei bevorzugt mindestens ein Gehäuse zum Begrenzen eines Aufnahmeraums auf, wobei das Gehäuse zumindest einerseits eine Kontaktoberfläche zur Anordnung der Körperkontakteinrichtung aufweist, wobei die Kontaktoberfläche eine mittels eines Verschlussteils einer Ventileinrichtung verschließbare Durchgangsöffnung aufweist, wobei die Ventileinrichtung zumindest mehrheitlich im Inneren des Aufnahmeraums angeordnet ist, wobei eine Feder zum Federkraftbeaufschlagen des Verschlussteils vorgesehen ist, wobei das Verschlussteil durch die Feder in eine die Durchgangsöffnung verschließende Position überführbar ist, und wobei die Durchgangsöffnung Bestandteil einer basiseinrichtungsseitigen Fluidleitung ist, wobei mindestens eine Pumpeinrichtung zum Druckbeaufschlagen des Arbeitsfluids, insbesondere Luft, vorgesehen ist, wobei die Pumpeinrichtung mit der Fluidleitung gekoppelt ist.

Gemäß einer bevorzugten Ausführungsform der vorliegenden Erfindung ist mindestens eine optische Signalquelle als Bestandteil des Gehäuses der Basiseinrichtung oder innerhalb des Gehäuses der Basiseinrichtung vorgesehen und/oder mindestens ein optischer Sensor ist als Bestandteil des Gehäuses der Basiseinrichtung oder innerhalb des Gehäuses der Basiseinrichtung vorgesehen. Die Pumpeinrichtung wird bevorzugt in Abhängigkeit von Signalen oder Daten der Sensoreinrichtung gesteuert oder ist in Abhängigkeit von Signalen oder Daten der Sensoreinrichtung steuerbar, wobei die Signale oder Daten von der Sensoreinrichtung in Abhängigkeit von detektierten optischen Signalen erzeugbar sind, welche aus optischen Signale resultieren, welche von der optischen Signalquelle emittiert werden. Weiterhin ist es möglich, dass eine Prozessoreinrichtung bzw. Steuereinrichtung Teil der Basiseinrichtung ist. Die Prozessoreinrichtung bzw. Steuereinrichtung übernimmt dabei bevorzugt die Aufbereitung und/oder Auswertung der Sensordaten und/oder eventueller Ventilsteuerungen und/oder die Pumpensteuerung. Diese Lösung ist vorteilhaft, da so alle oder zumindest die meisten Aktuatoren bzw. aktiven Einrichtungen, Elemente und/oder Komponenten bevorzugt Teil einer kompakten Einheit sind.

Die oben genannte Aufgabe wird ebenfalls durch eine Körperkontakteinrichtung zur Verwendung in einer erfindungsgemäßen Vorrichtung gelöst. Die Körperkontakteinrichtung weist dabei bevorzugt mindestens einen mit einem Arbeitsfluid, insbesondere Luft, beaufschlagbaren Druckapplikator, insbesondere Manschette, zur gesteuerten Druckbeaufschlagung eines Körperteils, insbesondere eines oder mehrerer Finger, auf und der Druckapplikator gibt bevorzugt eine Positionierung des Körperteils an der Körperkontakteinrichtung vor. Bevorzugt ist mindestens ein Dichtungselement zum lösbaren Ankoppeln der Körperkontakteinrichtung an einer Arbeitsfluidbereitstellungseinrichtung, insbesondere eine Basiseinrichtung gemäß Anspruch 10, vorgesehen, wobei die Körperkontakteinrichtung bevorzugt eine sich zwischen dem Dichtungselement und dem Druckapplikator erstreckende Fluidleitung aufweist, wobei die Körperkontakteinrichtung besonders bevorzugt eine zapfenförmige Auslenkeinrichtung aufweist, wobei die zapfenförmige Auslenkeinrichtung vollumfänglich und entlang ihrer Längserstreckungsrichtung zumindest abschnittsweise oder zumindest zeitweise von dem Dichtungselement umschlossen ist. Diese Lösung ist vorteilhaft, da die Position der Hand bzw. des oder der Finger unmittelbar vorgegeben ist. Ferner kann die Körperkontakteinrichtung sehr günstig ausgeführt sein und eignet sich somit zur kostengünstigen Einmalverwendung.

Mindestens ein Lichtleiter zum Zuführen von Strahlung zu dem Körperteil und/oder zum Bereitstellen von Strahlung an eine Sensoreinrichtung ist gemäß einer weiteren bevorzugten Ausführungsform der vorliegenden Erfindung vorgesehen, wobei der durch die Fluidleitung fließende Fluidstrom, insbesondere der Druck des Fluidstroms, in Abhängigkeit von der durch den Lichtleiter geleiteten Strahlung steuerbar ist. Diese Ausführungsform ist vorteilhaft, da das zu untersuchende Körperteil nicht an der Basiseinrichtung, sondern an der daran angeordneten Körperkontakteinrichtung angelegt werden kann. Somit erfolgt kein oder nur ein geringer Kontakt zwischen der Basiseinrichtung und dem Körperteil, wodurch eine Kontamination der Basiseinrichtung begrenzt ist.

Die obigen Vorrichtungen können insbesonder zur kontinuierlichen nichtinvasiven Messung des arteriellen Blutdrucks verwendet werden. Das Verfahren umfasst dabei bevorzugt die Schritte: Bereitstellen einer Vorrichtung nach einem der Ansprüche 1 bis 9, wobei die Basiseinrichtung und die Körperkontakteinrichtung in einer Betriebskonfiguration miteinander gekoppelt sind, Positionierung eines Körperteils, insbesondere eines Fingers oder mehrerer Finger, in dem Druckapplikator, Erzeugen eines optischen Signals mittels einer optischen Signalquelle, wobei die Signalquelle Bestandteil der Basiseinrichtung oder der Körperkontakteinrichtung ist, wobei das optische Signal in den Körperteil eingekoppelt wird, Erfassen des optischen Signals nach der Einkopplung in das Körperteil, insbesondere nach einer Streuung und/oder Reflexion durch das Körperteil, mittels einer optischen Sensoreinrichtung, wobei die optische Sensoreinrichtung Bestandteil der Basiseinrichtung oder der Körperkontakteinrichtung ist, Erzeugen eines Signals oder Daten durch die optische Sensoreinrichtung, wobei das Signal oder die Daten das erfasste optische Signal repräsentieren, Einstellen eines mittels des Druckapplikators auf den Körperteil ausgeübten Drucks in Abhängigkeit von dem durch die optische Sensoreinrichtung erzeugten Signal oder den erzeugten Daten.

Gemäß einer weiteren bevorzugten Ausführungsform der vorliegenden Erfindung sind die Signalquelle, die Sensoreinrichtung und die Pumpeinrichtung Bestandteil der Basiseinrichtung, wobei das durch die Signalquelle erzeugte optische Signal von der Basiseinrichtung durch die Körperkontakteinrichtung hindurch zu dem Körperteil geleitet wird und wobei die von dem Körperteil gestreute und/oder reflektierte Strahlung durch die Körperkontakteinrichtung hindurch zu der Basiseinrichtung geleitet wird und wobei das von der Pumpeinrichtung geförderte Arbeitsfluid Luft ist, wobei die Luft aus der Basiseinrichtung hinein durch die Fluidleitung in der Körperkontakteinrichtung zu dem Druckapplikator gefördert wird. Durch das Zusammenwirken der Sensoreinrichtung, der Signalquelle und der Druckerzeugung/Druckeinstellung in dem Druckapplikator, insbesondere Manschette, insbesondere mittels der Pumpeinrichtung und/oder einer gesteuerten Ventileinrichtung, wird eine Messung nach der Vascular Unloading Technique ermöglicht.

Weitere Vorteile, Ziele und Eigenschaften der vorliegenden Erfindung werden anhand nachfolgender Beschreibung anliegender Zeichnungen erläutert, in welchen beispielhaft die erfindungsgemäße Vorrichtung oder Bestandteile der erfindungsgemäßen Vorrichtung dargestellt ist. Bauteile oder Elemente, die in der erfindungsgemäßen Vorrichtung oder dem erfindungsgemäßen Verfahren bevorzugt eingesetzt werden und/oder welche in den Figuren wenigstens im Wesentlichen hinsichtlich ihrer Funktion übereinstimmen, können hierbei mit gleichen Bezugszeichen gekennzeichnet sein, wobei diese Bauteile oder Elemente nicht in allen Figuren beziffert oder erläutert sein müssen bzw. durchgeführt.

Darin zeigen:
- Fig. 1a-d: ein schematisches Beispiel einer erfindungsgemäßen Vorrichtung, deren Bestandteile (Basiseinrichtung und Körperkontakteinrichtung) und schematisch die Anordnung eines Körperteils an der Vorrichtung;
- Fig. 2a: einen Leitungsabschnitt einer Körperkontakteinrichtung mit daran fix angeordnetem Dichtungselement und fix angeordneter Auslenkeinrichtung,
- Fig. 2b: einen Leitungsabschnitt einer Körperkontakteinrichtung, wobei an dem Leitungsabschnitt ein Dichtungselement angeordnet ist und eine beweglich angeordnete Auslenkeinrichtung dichtend mit dem Dichtungselement zusammenwirkt. Das Dichtungselement bildet somit zwei zueinander beabstandete Dichtungswirkflächen aus, wobei eine Dichtungswirkfläche zum Zusammenwirken mit der Auslenkeinrichtung ausgebildet ist und eine Dichtungswirkfläche zum Zusammenwirken mit einer Kontaktfläche einer Basiseinrichtung ausgebildet ist,
- Fig. 3a: eine erste Ventileinrichtung, wobei diese Ventileinrichtung zumindest teilweise unmittelbar in einem Ventilgehäuse angeordnet ist, wobei das Ventilgehäuse im Gehäuse der Basiseinrichtung angeordnet oder ausgebildet ist,
- Fig. 3b: eine zweite Ventileinrichtung, wobei diese Ventileinrichtung zumindest teilweise in der Basiseinrichtung angeordnet oder ausgebildet ist,
- Fig. 4a: die in Fig. 2b gezeigte Anordnung in Verbindung mit der in Fig. 3b gezeigten Anordnung in einem ersten Zustand, in dem sich die Dichtung, insbesondere der Kragen, verformt und
- Fig. 4b: die in Fig. 2b gezeigte Anordnung in Verbindung mit der in Fig. 3b gezeigten Anordnung in einem zweiten Zustand, in dem sich die Dichtung, insbesondere der Kragen, und die Federn verformt haben, wodurch eine Fluidleitung zwischen der Basiseinrichtung und der Körperkontakteinrichtung geöffnet bzw. freigegeben bzw. ausgebildet wird,
- Fig. 5a: die in Fig. 2a gezeigte Anordnung in Verbindung mit der in Fig. 3b gezeigten Anordnung in einem ersten Zustand, d.h. in einem entkoppelten Zustand, und
- Fig. 5b: die in Fig. 5a gezeigten Komponenten in einem zweiten Zustand, wobei in diesem zweiten Zustand die Dichtung, insbesondere der Kragen, verformt ist und die Auslenkeinrichtung das Verschlussteil entgegen der Federkraft auslenkt, und
- Fig. 6: schematisch die in Fig. 5a gezeigte Anordnung in einer perspektivischen Darstellung und in einer perspektivischen Schnittdarstellung.

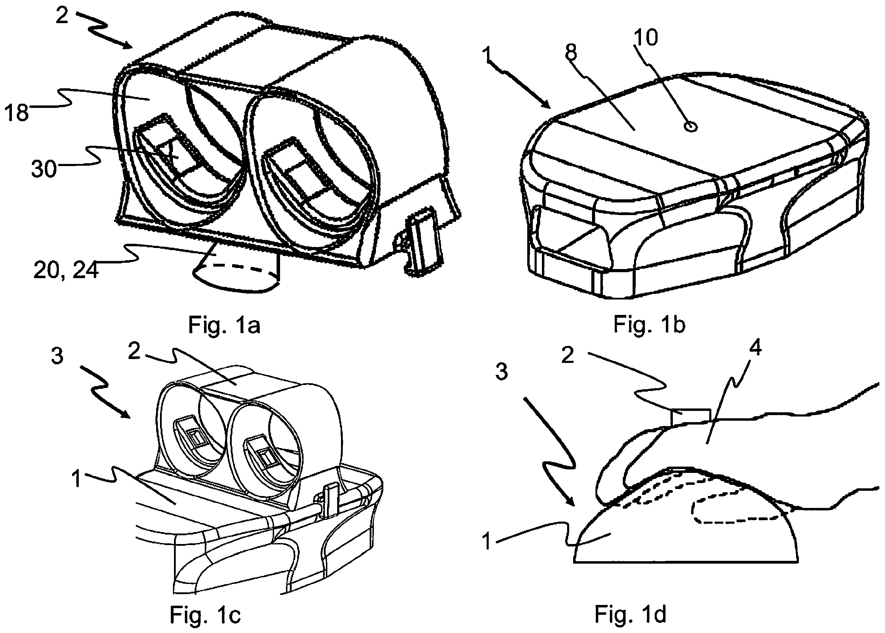

Fig. 1a zeigt ein Beispiel einer erfindungsgemäßen Körperkontakteinrichtung 2. Diese Körperkontakteinrichtung 2 weist einen Druckapplikator 18, insbesondere eine Manschette bzw. Druckmanschette, auf. Der Druckapplikator steht in einem Betriebszustand über eine oder mehrere Fluidleitungen mit einer in einer Basiseinrichtung 1 (vgl. Fig. 1b) angeordneten Pumpeinrichtung 1 in funktionaler, insbesondere fluidischer, Verbindung. Das Bezugszeichen 30 kennzeichnet rein schematisch eine Lichtkopplungsfläche zum Ein- bzw. Auskoppeln von Licht aus einem bzw. in einen Lichtleiter zur Bestrahlung eines Körperteils 4 (vgl. Fig. 1d) und/oder zur Zuführung von Licht, das vom Körperteil reflektiert und/oder gestreut wurde, zu einer Sensoreinrichtung. Alternativ kann die Vorrichtung auch so ausgeführt werden, dass eine Lichtquelle bzw. ein Lichtdetektor zum direkten Ein- bzw. Auskoppeln von Licht aus dem bzw. in den Körperteil in der Körperkontakteinrichtung 2 oder Basiseinrichtung 1 ohne zwischengeschalteten Lichtleiter vorgesehen ist.

Das Bezugszeichen 20 kennzeichnet ein Dichtungselement bzw. Dichtung bzw. Dichteinrichtung, das bevorzugt einen glockenförmigen oder (wie dargestellt) konusförmigen Kragen 24 aufweist.

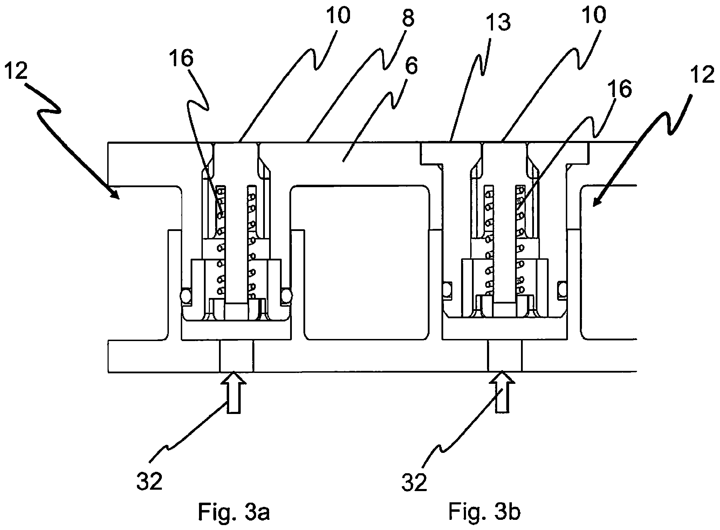

Das Dichtungselement 20 ist um ein Verschlussteil 10 einer Ventileinrichtung 12 (vgl. Fig. 3a und 3b) herum auf einer Oberfläche 8 der Basiseinrichtung 1 anordenbar.

Fig. 1c zeigt eine schematische Darstellung der erfindungsgemäßen Vorrichtung 3.

In Fig. 1d ist die erfindungsgemäße Vorrichtung 3 schematisch mit einer daran angeordneten menschlichen Hand 4 gezeigt.

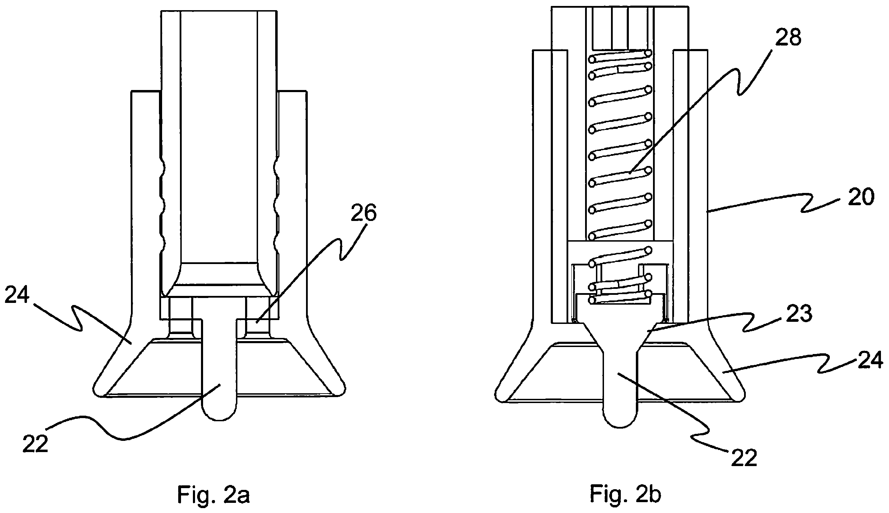

Fig. 2a zeigt ein Fluidleitungselement der Körperkontakteinrichtung 2, das zumindest abschnittsweise von dem Dichtungselement 20 umgeben ist. Das Dichtungselement 20 weist dabei mindestens oder genau eine Durchgangsöffnung 26 auf. Die Durchgangsöffnung/en 26 ist/sind bevorzugt stets geöffnet bzw. unverschlossen. Das Bezugszeichen 22 kennzeichnet dabei eine Auslenkeinrichtung zur Umpositionierung des Verschlussteils 10.

Fig. 2b zeigt ein alternatives Fluidleitungselement der Körperkontakteinrichtung 1, wobei die Auslenkeinrichtung 22 über eine Feder 28 mit einer Dichtfläche 23 an eine bevorzugt zentrale Durchgangsöffnung des Dichtungselements 20 anpressbar ist. Das Dichtungselement 20 weist ebenfalls einen konusförmigen Kragen 24 auf. Bevorzugt weist das Dichtungselement 20 hierbei genau eine Durchgangsöffnung auf.

Fig. 3a zeigt eine erste Ventileinrichtung 12. Die Ventileinrichtung 12 weist ein Verschlussteil 10 auf, das mit der Oberfläche 8 der Basiseinrichtung 1 bzw. des Gehäuses 6 der Basiseinrichtung bündig abschließt. Das Bezugszeichen 32 kennzeichnet eine Druckluftzuführung, insbesondere ausgehend von einer Pumpeinrichtung.

Fig. 3b zeigt eine zweite Ventileinrichtung 12. Gemäß dieser Ventileinrichtung 12 ist ein Ventilgehäuse 13 vorgesehen, mittels dem die Ventileinrichtung 12 mit dem Gehäuse 6 der Basiseinrichtung 1 gekoppelt ist.

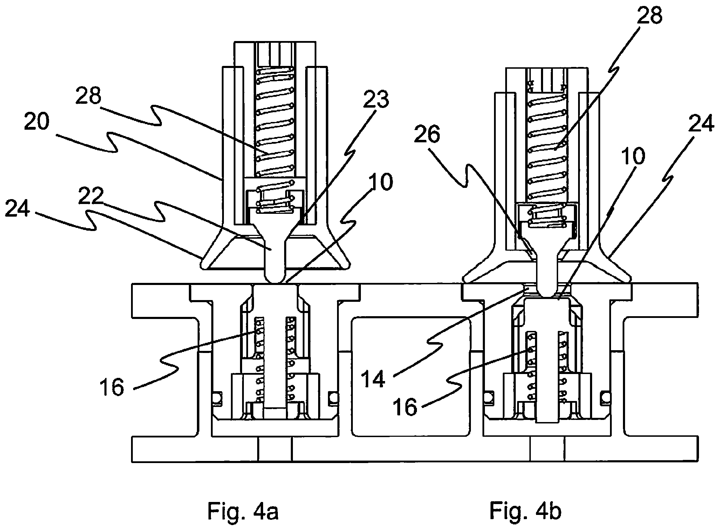

Fig. 4a zeigt die Ventileinrichtung aus Fig. 3b und die Anordnung aus Fig. 2b in einem ersten Zustand. Das Dichtelement 20 weist bereits eine erste Verformung auf und die Auslenkeinrichtung 22 bewirkt bereits eine Umpositionierung des Verschlussteils 10. Die Dichtfläche 23 dichtet jedoch in diesem Zustand noch ab.

In Fig. 4b ist die Verformung des Dichtungselements 20, insbesondere des Kragens 24, weiter vorangeschritten, wodurch sich das Leitungselement der Körperkontakteinrichtung 2 der Basiseinrichtung 1 weiter genähert hat. Nach einer definierten Auslenkung des Verschlussteils 10 erfolgt dann eine Relativbewegung des Dichtungselements 20 gegenüber der Auslenkeinrichtung 22, wodurch die Durchgangsöffnung 26 freigegeben wird.

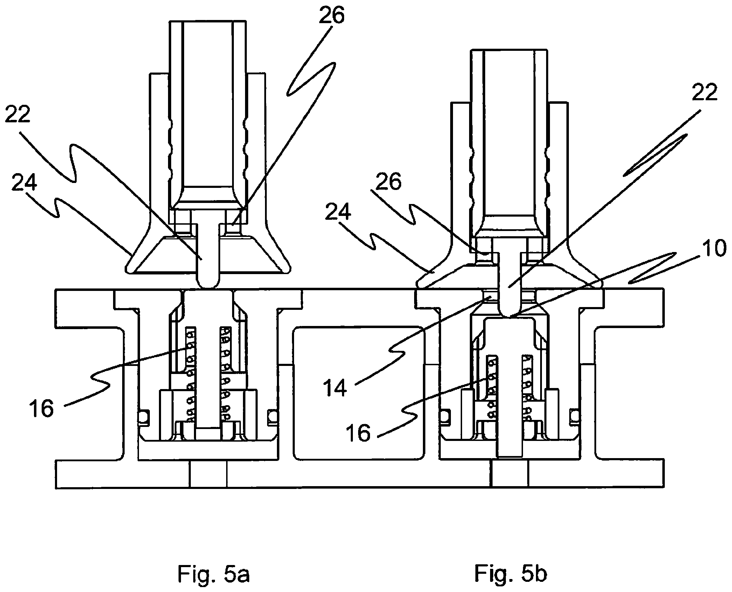

Fig. 5a zeigt die Anordnung aus Fig. 2 und die Ventileinrichtung 12 aus Fig. 3b. Hierbei kann das Dichtungselement 20 lediglich eine Verformung erfahren und das Verschlussteil 10 ausgelenkt werden. Die Auslenkung des Verschlussteils 10 hängt dabei bevorzugt direkt mit einer Verformung des Dichtungselements 20 zusammen. Dieser Zusammenhang ist durch Fig. 5b dargestellt.

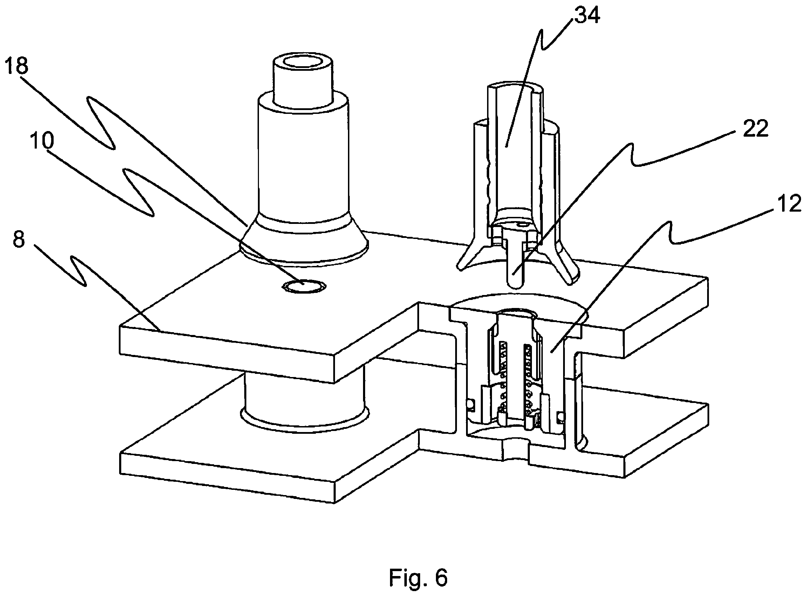

In Fig. 6 ist die in Fig. 5a gezeigte Anordnung in einer perspektivischen Darstellung und in einer Schnittdarstellung gezeigt.

Somit bezieht sich das vorliegende Ausführungsbeispiel auf eine Vorrichtung 3 zum Erfassen von Vitalparametern. Diese Vorrichtung 3 weist auf: Eine Basiseinrichtung mit einer Pumpeinrichtung zum Druckbeaufschlagen eines Arbeitsfluids und eine Körperkontakteinrichtung 2 zum definierten Anordnen eines Körperteils 4, insbesondere eines Fingers, wobei die Basiseinrichtung 1 und die Körperkontakteinrichtung 2 zum Ausbilden einer Betriebskonfiguration miteinander lösbar koppelbar sind, wobei die Basiseinrichtung 1 ein Gehäuse 6 zum Begrenzen eines Aufnahmeraums aufweist, wobei das Gehäuse 6 eine Kontaktoberfläche 8 zur Anordnung der Körperkontakteinrichtung 2 aufweist, wobei die Kontaktoberfläche 8 eine mittels eines Verschlussteils 10 einer Ventileinrichtung 12 verschließbare Durchgangsöffnung 14 aufweist, wobei die Ventileinrichtung 12 zumindest überwiegend im Inneren des Aufnahmeraums angeordnet ist, wobei eine Feder 16 zum Federkraftbeaufschlagen des Verschlussteils 10 vorgesehen ist, wobei das Verschlussteil 10 durch die Feder 16 in eine die Durchgangsöffnung 14 verschließende Position überführbar ist, und wobei die Durchgangsöffnung 14 Bestandteil einer basiseinrichtungsseitigen Fluidleitung ist, wobei die Körperkontakteinrichtung 2 mindestens einen mit dem Arbeitsfluid beaufschlagbaren Druckapplikator 18 zur gesteuerten Druckbeaufschlagung des Körperteils 4 aufweist und wobei die Körperkontakteinrichtung 2 mindestens ein Dichtungselement 20 zum Erzeugen einer Fluidverbindung mit der Basiseinrichtung 1 aufweist, wobei die Körperkontakteinrichtung 2 zwischen dem Dichtungselement 20 und dem Druckapplikator 18 eine körperkontakteinrichtungsseitige Fluidleitung aufweist, wobei das Dichtungselement 20 zum Umschließen der Durchgangsöffnung 14 an die Kontaktoberfläche 8 anpressbar ist, wobei die Körperkontakteinrichtung 2 eine Auslenkeinrichtung 22 zum Auslenken des Verschlussteils 10 aufweist, wobei die Auslenkeinrichtung 22 in der Betriebskonfiguration das Verschlussteil 10 entgegen der Federkraft und unter Ausbildung der Fluidverbindung auslenkt.

### Bezugszeichenliste

- 1: Basiseinrichtung
- 2: Körperkontakteinrichtung
- 3: Vorrichtung
- 4: Körperteil (erstes Körperteil / erster Finger)
- 5: zweites Körperteil (zweiter Finger)
- 6: Gehäuse
- 8: Kontaktoberfläche
- 10: Verschlussteil
- 12: Ventileinrichtung
- 13: Ventilgehäuse
- 14: Durchgangsöffnung
- 16: Feder
- 18: Druckapplikator / Manschette
- 20: Dichtungselement
- 22: Auslenkeinrichtung
- 23: Dichtfläche zwischen Dichtung und Auslenkeinrichtung
- 24: Kragen
- 26: Fluiddurchlass / Durchgangsöffnung (im Dichtungselement)
- 28: Feder (zur Auslenkung der Auslenkeinrichtung)
- 30: Lichtkopplungsfläche
- 32: Druckluftzuführung
- 34: Fluidleitung in der Körperkontakteinrichtung

## Patentansprüche

1. Vorrichtung zum Erfassen von Vitalparametern,
mindestens aufweisend
- eine Basiseinrichtung (1) aufweisend Mittel zur kontrollierten Druckbeaufschlagung eines Arbeitsfluids und
- eine Körperkontakteinrichtung (2) zum definierten Anordnen eines Körperteils (4), insbesondere eines Fingers,
wobei die Basiseinrichtung (1) und die Körperkontakteinrichtung (2) zum Ausbilden einer Betriebskonfiguration lösbar miteinander koppelbar sind,
wobei die Basiseinrichtung (1) mindestens ein Gehäuse (6) zum Begrenzen eines Aufnahmeraums aufweist,
wobei das Gehäuse (6) eine Kontaktoberfläche (8) zur Anordnung der Körperkontakteinrichtung (2) aufweist,
wobei die Kontaktoberfläche (8) eine mittels eines Verschlussteils (10) einer Ventileinrichtung (12) verschließbare Durchgangsöffnung (14) aufweist,
wobei die Ventileinrichtung (12) zumindest überwiegend im Inneren des Aufnahmeraums angeordnet ist
wobei eine Feder (16) zum Federkraftbeaufschlagen des Verschlussteils (10) vorgesehen ist,
wobei das Verschlussteil (10) durch die Feder (16) in eine die Durchgangsöffnung (14) verschließende Position überführbar ist, und
wobei die Durchgangsöffnung (14) Bestandteil einer basiseinrichtungsseitigen Fluidleitung ist,
wobei die Körperkontakteinrichtung (2) mindestens einen mit dem Arbeitsfluid beaufschlagbaren Druckapplikator (18) zur gesteuerten Druckbeaufschlagung des Körperteils (4) aufweist und
wobei die Körperkontakteinrichtung (2) mindestens ein Dichtungselement (20) zum Erzeugen einer Fluidverbindung mit der Basiseinrichtung (1) aufweist,
wobei die Körperkontakteinrichtung (2) zwischen dem Dichtungselement (20) und dem Druckapplikator (18) eine körperkontakteinrichtungsseitige Fluidleitung aufweist,
wobei das Dichtungselement (20) zum Umschließen der Durchgangsöffnung (14) an die Kontaktoberfläche (8) anpressbar ist,
wobei die Körperkontakteinrichtung (2) eine Auslenkeinrichtung (22) zum Auslenken des Verschlussteils (10) aufweist, und
wobei die Auslenkeinrichtung (22) in der Betriebskonfiguration das Verschlussteil (10) entgegen der Federkraft und unter Ausbildung der Fluidverbindung auslenkt.

2. Vorrichtung nach Anspruch 1,
**dadurch gekennzeichnet, dass**
das Verschlussteil (10) einen Teil der Kontaktoberfläche (8) des Gehäuses (6) ausbildet, wobei der durch das Verschlussteil (10) ausgebildete Teil der Kontaktoberfläche (8) in einer entkoppelten Konfiguration bündig mit den umliegenden Oberflächenanteilen der Kontaktoberfläche (8) abschließt.

3. Vorrichtung nach Anspruch 1 oder 2,
**dadurch gekennzeichnet, dass**
das Dichtungselement (20) einen konischen oder glockenförmigen Kragen (24) aufweist, wobei der konische oder glockenförmige Kragen (24) die Auslenkeinrichtung (22) zumindest zeitweise und zumindest abschnittsweise in Umfangsrichtung umschließt, wobei die Auslenkeinrichtung (22) im Zentrum des konischen oder glockenförmigen Kragens (24) angeordnet ist,
wobei der konische oder glockenförmige Kragen (24) im Bereich eines ersten Endes in radialer Richtung weiter von der Auslenkeinrichtung (22) beabstandet ist als im Bereich eines zweiten Endes, wobei das erste Ende des konischen oder glockenförmigen Kragens (24) zum Kontaktieren der Kontaktoberfläche (8) bestimmt ist.

4. Vorrichtung nach einem der vorangegangenen Ansprüche, **dadurch gekennzeichnet, dass**
die Auslenkeinrichtung (22) fix gegenüber dem Dichtungselement (20) positioniert ist und zwischen dem konischen oder glockenförmigen Kragen (24) des Dichtungselements (20) und der Auslenkeinrichtung (22) mindestens ein Fluiddurchlass (26) ausgebildet ist und bevorzugt mehrere Fluiddurchlässe (26) ausgebildet sind.

5. Vorrichtung nach einem der vorangegangenen Ansprüche 1 bis 3,
**dadurch gekennzeichnet, dass**
die Auslenkeinrichtung (22) gegenüber dem Dichtungselement (20) beweglich angeordnet ist, wobei das Dichtungselement (20) eine Durchgangsöffnung (26) ausbildet, wobei die Durchgangsöffnung (26) fluiddicht durch die Auslenkeinrichtung (22) verschließbar ist, wobei die Auslenkeinrichtung (22) die Durchgangsöffnung (26) des Dichtungselements (20) in einer Nichtgebrauchskonfiguration verschließt und in der Betriebskonfiguration frei gibt.

6. Vorrichtung nach Anspruch 5,
**dadurch gekennzeichnet, dass**
eine Feder (28) zum Auslenken der Auslenkeinrichtung (30) vorgesehen ist, wobei die Auslenkeinrichtung (30) geradlinig auslenkbar ist, wobei die Feder (28) in der Betriebskonfiguration infolge der geradlinigen Auslenkung der Auslenkeinrichtung (22) stärker ausgelenkt, insbesondere gestaucht, ist als in der Nichtgebrauchskonfiguration.

7. Vorrichtung nach Anspruch 6,
**dadurch gekennzeichnet, dass**
die Auslenkung der Auslenkeinrichtung (22) durch einen Anschlag begrenzt ist,
wobei die Durchgangsöffnung (26) infolge der Verformung des konischen oder glockenförmigen Kragens (24) betragsmäßig näher an die Basiseinrichtung (1) heranbewegbar ist als die Auslenkeinrichtung (22) auslenkbar ist oder als das Verschlussteil (10) der Ventileinrichtung (12) der Basiseinrichtung (1) ausgelenkt wird oder auslenkbar ist.

8. Vorrichtung nach Anspruch 7, **dadurch gekennzeichnet, dass**
die Auslenkung der Auslenkeinrichtung (22) und die Auslenkung des Verschlussteils (10) der Ventileinrichtung (12) der Basiseinrichtung (1) in Summe der sich infolge der Verformung des konischen oder glockenförmigen Kragens (24) resultierenden Annäherung der Durchgangsöffnung des Dichtungselements (20) an die Kontaktoberfläche (8) entsprechen.

9. Vorrichtung nach einem der vorangegangenen Ansprüche,
**dadurch gekennzeichnet, dass**
basiseinrichtungsseitig mindestens eine Pumpeinrichtung zum Druckbeaufschlagen des Arbeitsfluids, insbesondere Luft, vorgesehen ist, wobei die Pumpeinrichtung mit der Fluidleitung gekoppelt ist,
wobei mindestens eine optische Signalquelle zur Aussendung optischer Signale in Gewebe des Körperteils (4) und eine optische Sensoreinrichtung zum Erfassen resultierender optischer Signale aus dem Gewebe des Körperteils (4) vorgesehen sind, wobei mindestens ein Funktionsparameter der Pumpeinrichtung und/oder des Druckapplikators in Abhängigkeit der erfassten resultierenden optischen Signale regelbar ist.

10. Basiseinrichtung (1) zur Verwendung in einer Vorrichtung gemäß einem der vorangegangenen Ansprüche, wobei die Basiseinrichtung (1) mindestens ein Gehäuse (6) zum Begrenzen eines Aufnahmeraums aufweist,
wobei das Gehäuse (6) eine Kontaktoberfläche (8) zur Anordnung der Körperkontakteinrichtung (2) aufweist,
wobei die Kontaktoberfläche (8) eine mittels eines Verschlussteils (10) einer Ventileinrichtung (12) verschließbare Durchgangsöffnung (14) aufweist,
wobei die Ventileinrichtung (12) zumindest überwiegend im Inneren des Aufnahmeraums angeordnet ist,
wobei eine Feder (16) zum Federkraftbeaufschlagen des Verschlussteils (10) vorgesehen ist,
wobei das Verschlussteil (10) durch die Feder (16) in eine die Durchgangsöffnung (14) verschließende Position überführbar ist,
und
wobei die Durchgangsöffnung (14) Bestandteil einer basiseinrichtungsseitigen Fluidleitung ist,
wobei mindestens eine Pumpeinrichtung zum Druckbeaufschlagen des Arbeitsfluids, insbesondere Luft, vorgesehen ist, wobei die Pumpeinrichtung mit der Fluidleitung gekoppelt ist.

11. Basiseinrichtung (1) nach Anspruch 10,
**dadurch gekennzeichnet, dass**
mindestens eine optische Signalquelle zur Aussendung optischer Signale in Gewebe des Körperteils (4) als Bestandteil des Gehäuses (6) der Basiseinrichtung (1) oder innerhalb des Gehäuses (6) der Basiseinrichtung (1) vorgesehen ist
und/oder
mindestens ein optischer Sensor zum Erfassen resultierender optischer Signale aus dem Gewebe des Körperteils (4) als Bestandteil des Gehäuses (6) der Basiseinrichtung (1) oder innerhalb des Gehäuses (6) der Basiseinrichtung (1) vorgesehen ist,
wobei mindestens ein Funktionsparameter der Pumpeinrichtung und/oder des Druckapplikators in Abhängigkeit der erfassten resultierenden optischen Signale regelbar ist.

12. Körperkontakteinrichtung (2) zur Verwendung in einer Vorrichtung gemäß einem der vorangegangenen Ansprüche 1 bis 9,
wobei mindestens ein mit einem Arbeitsfluid, insbesondere Luft, beaufschlagbarer Druckapplikator (18), insbesondere Manschette, zur gesteuerten Druckbeaufschlagung eines Körperteils (4), insbesondere eines oder mehrerer Finger, vorgesehen ist und der Druckapplikator (18) eine Positionierung des Körperteils (4) an der Körperkontakteinrichtung (2) vorgibt,
wobei mindestens ein Dichtungselement (20) zum lösbaren Ankoppeln der Körperkontakteinrichtung (2) an einer Arbeitsfluidbereitstellungseinrichtung, insbesondere eine Basiseinrichtung gemäß Anspruch 10, vorgesehen ist,
wobei die Körperkontakteinrichtung (2) eine sich zwischen dem Dichtungselement (20) und dem Druckapplikator (18) erstreckende Fluidleitung aufweist,
wobei die Körperkontakteinrichtung (2) eine zapfenförmige Auslenkeinrichtung (22) aufweist, wobei die zapfenförmige Auslenkeinrichtung (22) vollumfänglich und entlang ihrer Längserstreckungsrichtung zumindest abschnittsweise oder zumindest zeitweise von dem Dichtungselement (20) umschlossen ist.

13. Körperkontakteinrichtung nach Anspruch 12,
**dadurch gekennzeichnet, dass**
mindestens ein Lichtleiter zum Zuführen von Strahlung zu dem Körperteil (4) und/oder zum Bereitstellen von Strahlung an eine Sensoreinrichtung vorgesehen ist.

## Claims

1. A device for recording vital signs,
comprising at least
- a base device (1) comprising means for controlled pressurization of a working fluid and
- a body contact device (2) for defined positioning of a body part (4), in particular a finger,
wherein the base device (1) and the body contact device (2) can be releasably coupled to each other to form an operating configuration,
wherein the base device (1) com prises at least one housing (6) for delim iting a receiving space,
said housing (6) comprising a contact surface (8) for disposing said body contact device (2),
wherein the contact surface (8) has a passage (14) which is closable by means of a closing member(10) of a valve device (12),
wherein the valve device (12) is arranged at least predominantly in the interior of the receiving space,
wherein a spring (16) is provided for spring-loading the closing member(10),
wherein the closing member(10) is moveable by the spring (16) into a position closing the passage (14),
and
wherein the passage (14) is a component of a fluid line on the base device side,
wherein the body contact device (2) comprises at least one pressure applicator (18), which can be acted upon by the working fluid, for controlled pressurization of the body part (4), and
wherein the body contact device (2) comprises at least one sealing element (20) for establishing a fluid connection to the base device (1),
wherein the body contact device (2) comprises a fluid line on the body contact device side between the sealing element (20) and the pressure applicator (18),
wherein the sealing element (20) is pressable against the contact surface (8) for enclosing the passage (14),
wherein the body contact device (2) comprises a deflecting means (22) for deflecting the closing member(10), and
wherein the deflecting means (22), in the operating configuration, deflects the closing member (10) against the spring force with formation of the fluid connection.

2. The device according to claim 1,
**characterized in that**
the closing member (10) forms a part of the contact surface (8) of the housing (6), wherein the part of the contact surface (8) formed by the closing member (10) terminates flush with the surrounding surface parts of the contact surface (8) in a decoupled configuration.

3. The device according to claim 1 or 2,
**characterized in that**
the sealing element (20) has a conical or bell-shaped collar (24), wherein the conical or bell-shaped collar (24) encloses the deflecting means (22) at least temporarily and at least in sections in the circumferential direction, wherein the deflecting means (22) is arranged in the center of the conical or bell-shaped collar (24),
wherein the conical or bell-shaped collar (24) is at a greater distance in the radial direction from the deflecting means (22) in the area of a first end than in the area of a second end, wherein the first end of the conical or bell-shaped collar (24) is determined for establishing contact with the contact surface (8).

4. The device according to any of the preceding claims,
**characterized in that**
the deflecting means (22) is fixedly positioned opposite the sealing element (20), and at least one fluid passage (26) is formed and preferably several fluid passages (26) are formed between the conical or bell-shaped collar (24) of the sealing element (20) and the deflecting means (22).

5. The device according to any of claims 1 to 3,
**characterized in that**
the deflecting means (22) is movably arranged with respect to the sealing element (20), wherein the sealing element (20) forms a passage (26), wherein the passage (26) is closable in a fluid-tight manner by the deflecting means (22), wherein the deflecting means (22) closes the passage (26) of the sealing element (20) in a non-use configuration and releases it in the operating configuration.

6. The device according to claim 5,
**characterized in that**
a spring (28) is provided for deflecting the deflecting means (30), wherein the deflecting means (30) is deflectable in a straight line, wherein the spring (28) is more strongly deflected, in particular compressed, in the operating configuration as a result of the straight deflection of the deflecting means (22) than in the non-use configuration.

7. The device according to claim 6,
**characterized in that**
the deflection of the deflecting means (22) is limited by a stop,
wherein the passage (26) can be moved closer to the base device (1), in terms of amount, as a result of the deformation of the conical or bell-shaped collar (24) than the deflecting means (22) or than the closing member(10) of the valve device (12) of the base device (1) is deflected or can be deflected.

8. The device according to claim 7,
**characterized in that**
the deflection of the deflecting means (22) and the deflection of the closing member (10) of the valve device (12) of the base device (1) in total correspond to the approach of the passage of the sealing element (20) toward the contact surface (8) due to the deformation of the conical or bell-shaped collar (24).

9. The device according to any of the preceding claims,
**characterized in that**
at least one pump device for pressurizing the working fluid, in particular air, is provided on the base device side, wherein the pump device is coupled to the fluid line,
wherein at least one optical signal source for transmitting optical signals into tissue of the body part (4) and an optical sensor device for detecting resulting optical signals from the tissue of the body part (4) are provided,
wherein at least one functional parameter of the pump device and/or of the pressure applicator can be regulated as a function of the resulting optical signals detected.

10. A base device (1) for use in a device according to any of the preceding claims, wherein
the base device (1) comprises at least one housing (6) for delimiting a receiving space,
wherein the housing (6) com prises a contact surface (8) for arranging said body contact device (2),
wherein the contact surface (8) has a passage (14) which is closable by means of a closing member (10) of a valve device (12),
wherein the valve device (12) is arranged at least predominantly in the interior of the receiving space,
wherein a spring (16) is provided for spring-loading the closing member(10),
wherein the closing member(10) is moveable by the spring (16) into a position closing the passage (14),
and
wherein the passage (14) is a component of a fluid line on the base device side,
wherein at least one pump device for pressurizing the working fluid, in particular air, is provided, wherein the pump device is coupled to the fluid line.

11. The base device (1) according to claim 10, **characterized in that**
at least one optical signal source for emitting optical signals in the tissue of the body part (4) is provided as part of the housing (6) of the base device (1) or within the housing (6) of the base device (1),
and/or
at least one optical sensor for detecting resulting optical signals from the tissue of the body part (4) is provided as part of the housing (6) of the base device (1) or within the housing (6) of the base device (1),
wherein at least one functional parameter of the pump device and/or of the pressure applicator can be regulated as a function of the resulting optical signals detected.

12. A body contact device (2) for use in a device according to any of preceding claims 1 to 9,
wherein
at least one pressure applicator (18), in particular a cuff, which can be acted upon by a working fluid, in particular air, is provided for the controlled application of pressure to a body part (4), in particular one or more fingers, and the pressure applicator (18) specifies the body part (4) to be positioned on the body contact device (2),
wherein at least one sealing element (20) is provided for releasably coupling the body contact device (2) to a working fluid supply device, in particular a base device according to claim 10,
wherein the body contact device (2) has a fluid line extending between the sealing element (20) and the pressure applicator (18),
wherein the body contact device (2) has a pin-shaped deflecting means (22), wherein the pin-shaped deflecting means (22) is completely and at least partially or at least temporarily enclosed by the sealing element (20) along its longitudinal extension direction.

13. The body contact device according to claim 12,
**characterized in that**
at least one light guide is provided for supplying radiation to the body part (4) and/or for providing radiation to a sensor device.

## Revendications

1. Dispositif de mesure de paramètres vitaux,
com prenant au moins
- un dispositif de base (1) comprenant des moyens pour l'application contrôlée d'une pression d'un fluide de travail et
- un dispositif de contact corporel (2) pour la disposition définie d'une partie du corps (4), plus particulièrement d'un doigt,
dans lequel le dispositif de base (1) et le dispositif de contact corporel (2) peuvent être couplés entre eux de manière amovible pour la formation d'une configuration de fonctionnement,
dans lequel le dispositif de base (1) comprend au moins un boîtier (6) pour la délimitation d'un espace de logement,
dans lequel le boîtier (6) comprend une surface de contact (8) pour la disposition du dispositif de contact corporel (2),
dans lequel la surface de contact (8) comprend une ouverture de passage (14) pouvant être fermée au moyen d'une partie de fermeture (10) d'un dispositif de soupape (12),
dans lequel le dispositif de soupape (12) est disposé au moins majoritairement à l'intérieur de l'espace de logements
dans lequel un ressort (16) est prévu pour l'application d'une force élastique sur la partie de fermeture (10),
dans lequel la partie de fermeture (10) peut être mise par le ressort (16) dans une position fermant l'ouverture de passage (14),
et
dans lequel l'ouverture de passage (14) fait partie d'une conduite de fluide du côté du dispositif de base,
dans lequel le dispositif de contact corporel (2) comprend au moins un applicateur de pression (18) pouvant être alimenté en fluide de travail, pour l'application d'une pression contrôlée sur la partie de corps (4) et
dans lequel le dispositif de contact corporel (2) comprend au moins un élément d'étanchéité (20) pour l'établissement d'une liaison fluidique avec le dispositif de base (1),
dans lequel le dispositif de contact corporel (2) comprend, entre l'élément d'étanchéité (20) et l'applicateur de pression (18), une conduite de fluide du côté du dispositif de contact corporel,
dans lequel l'élément d'étanchéité (20) peut être pressé contre la surface de contact (8) afin d'entourer l'ouverture de passage (14),
dans lequel le dispositif de contact corporel (2) comprend un dispositif de déviation (22) pour la déviation de la partie de fermeture (10) et
dans lequel le dispositif de déviation (22) dévie, dans la configuration de fonctionnement, la partie de fermeture (10) à l'encontre la force du ressort et en formant la liaison fluidique.

2. Dispositif selon la revendication 1,
**caractérisé en ce que**
la partie de fermeture (10) forme une partie de la surface de contact (8) du boîtier (6), dans lequel la partie de la surface de contact (8) constituée de la partie de fermeture (10) se termine, dans une configuration découplée, de manière alignée avec les parties de surface de la surface de contact (8) se trouvant autour.

3. Dispositif selon la revendication 1 ou 2,
**caractérisé en ce que**
l'élément d'étanchéité (20) comprend un col conique ou en forme de cloche (24), dans lequel le col conique ou en forme de cloche (24) entoure le dispositif de déviation (22) au moins temporairement et au moins à certains endroits dans la direction circonférentielle, dans lequel le dispositif de déviation (22) est disposé au centre du col conique ou en forme de cloche (24),
dans lequel le col conique ou en forme de cloche (24) est en outre plus éloignée, au niveau d'une première extrémité, dans la direction radiale, du dispositif de déviation (22) qu'au niveau d'une deuxième extrémité, dans lequel la première extrémité du col conique ou en forme de cloche (24) est conçue pour une mise en contact avec la surface de contact (8).

4. Dispositif selon l'une des revendications précédentes,
**caractérisé en ce que**
le dispositif de déviation (22) est positionné de manière fixe par rapport à l'élément d'étanchéité (20) et, entre le col conique ou en forme de cloche (24) de l'élément d'étanchéité (20) et le dispositif de déviation (22), est formé au moins un passage de fluide (26), et de préférence plusieurs passages de fluide (26) sont formés.

5. Dispositif selon l'une des revendications 1 à 3,
**caractérisé en ce que**
le dispositif de déviation (22) est disposé de manière mobile par rapport à l'élément d'étanchéité (20), dans lequel l'élément d'étanchéité (20) forme une ouverture de passage (26), dans lequel l'ouverture de passage (26) peut être fermée de manière étanche aux fluides par le dispositif de déviation (22), dans lequel le dispositif de déviation (22) ferme l'ouverture de passage (26) de l'élément d'étanchéité (20) dans une configuration de non-utilisation et la libère dans la configuration de fonctionnement.

6. Dispositif selon la revendication 5,
**caractérisé en ce que**
un ressort (28) est prévu pour la déviation du dispositif de déviation (30), dans lequel le dispositif de déviation (30) peut être dévié en ligne droite, dans lequel le ressort (28), est dévié plus fortement, à la suite de la déviation en ligne droite du dispositif de déviation (22), dans la configuration de fonctionnement que dans la configuration de non-utilisation.

7. Dispositif selon la revendication 6,
**caractérisé en ce que**
la déviation du dispositif de déviation (22) est limitée par une butée,
dans lequel l'ouverture de passage (26) peut être déplacé, à la suite de la déformation du col conique ou en forme de cloche (24), plus près du dispositif de base (1) que le dispositif de déviation (22) peut être dévié ou que la partie de fermeture (10) du dispositif de soupape (12) du dispositif de base (1) est dévié ou peut être dévié.

8. Dispositif selon la revendication 7,
**caractérisé en ce que**
la déviation du dispositif de déviation (22) et la déviation de la partie de fermeture (10) du dispositif de soupape (12) du dispositif de base (1) correspondent, au total, à l'approche, résultant de la déformation du col conique ou en forme de cloche (24), de l'ouverture de passage de l'élément d'étanchéité (20) en direction de la surface de contact (8).

9. Dispositif selon l'une des revendications précédentes,
**caractérisé en ce que**
du côté du dispositif de base, est prévu au moins un dispositif de pompage pour l'application d'une pression à un fluide de travail, plus particulièrement de l'air, dans lequel le dispositif de pompage est couplé avec la conduite de fluide,
dans lequel au moins une source de signaux optiques est conçue pour l'émission de signaux optiques dans le tissu de la partie du corps (4) et un dispositif de capteur optique pour la mesure de signaux optiques qui en résultent hors du tissu de la partie du corps (4),
dans lequel au moins un paramètre de fonction du dispositif de pompage et/ou de l'applicateur de pression peut être régulé en fonction des signaux optiques détectés.

10. Dispositif de base (1) pour une utilisation dans un dispositif selon l'une des revendications précédentes,
dans lequel le dispositif de base (1) comprend au moins un boîtier (6) pour la délimitation d'un espace de logement,
dans lequel le boîtier (6) comprend une surface de contact (8) pour la disposition du dispositif de contact corporel (2),
dans lequel la surface de contact (8) comprend une ouverture de passage (14) pouvant être fermée au moyen d'une partie de fermeture (10) d'un dispositif de soupape (12),
dans lequel le dispositif de soupape (12) est disposé au moins majoritairement à l'intérieur de l'espace de logements
dans lequel un ressort (16) est prévu pour l'application d'une force élastique sur la partie de fermeture (10),
dans lequel la partie de fermeture (10) peut être mise par le ressort (16) dans une position fermant l'ouverture de passage (14),
et
dans lequel l'ouverture de passage (14) fait partie d'une conduite de fluide du côté du dispositif de base,
dans lequel au moins un dispositif de pompage est prévu pour l'application d'une pression au fluide de travail, plus particulièrement de l'air, dans lequel le dispositif de pompage est couplé avec la conduite de fluide.

11. Dispositif de base (1) selon la revendication 10,
**caractérisé en ce que**
au moins une source de signaux optiques est prévue pour l'émission de signaux optiques dans le tissu de la partie du corps (4) en tant que partie du boîtier (6) du dispositif de base (1) ou à l'intérieur du boîtier (6) du dispositif de base (1)
et/ou
au moins un capteur optique est prévu pour la détection des signaux optiques résultants sortant du tissu de la partie du corps (4) en tant que partie du boîtier (6) du dispositif de base (1) ou à l'intérieur du boîtier (6) du dispositif de base (1),
dans lequel au moins un paramètre de fonction du dispositif de pompage et/ou de l'applicateur de pression peut être régulé en fonction des signaux optiques détectés.

12. Dispositif de contact corporel (2) pour une utilisation dans un dispositif selon l'une des revendications 1 à 9,
dans lequel au moins un applicateur de pression (18), pouvant être alimenté avec un fluide de travail, plus particulièrement de l'air, plus particulièrement une manchette, est prévu pour l'application contrôlée d'une pression sur une partie du corps (4), plus particulièrement un ou plusieurs doigts et l'applicateur de pression (18) prédétermine un positionnement de la partie du corps (4) sur le dispositif de contact corporel (2),
dans lequel au moins un élément d'étanchéité (20) est prévu pour le couplage amovible du dispositif de contact corporel (2) à un dispositif de mise à disposition de fluide de travail, plus particulièrement un dispositif de base selon la revendication 10,
dans lequel le dispositif de contact corporel (2) comprend une conduite de fluide s'étendant entre l'élément d'étanchéité (20) et l'applicateur de pression (18),
dans lequel le dispositif de contact corporel (2) comprend un dispositif de déviation en forme de tenon (22),
dans lequel le dispositif de déviation en forme de tenon (22) est entouré, sur toute sa circonférence et le long de son extension longitudinale, au moins à certains endroits et au moins temporairement, par l'élément d'étanchéité (20).

13. Dispositif de contact corporel selon la revendication 12,
**caractérisé en ce que**
au moins une fibre optique est prévue pour l'introduction d'un rayonnement dans la partie du corps (4) et/ou pour la mise à disposition d'un rayonnement au niveau d'un dispositif de capteur.
